# DEMANDE DE BREVET EUROPEEN

(11) **EP 1 057 477 A1**
(43) Date de publication de la demande: **06.12.2000**
(21) Numéro de dépôt: 00401245.6
(22) Date de dépôt: 05.05.2000
(51) Int. Cl.: A61K 7/48, A61K 7/06, A61K 7/02

(54) **Composition sous forme d'émulsion huile-dans-l'eau contenant des fibrilles de cellulose, et ses utilisations notamment cosmétiques**

(30) Priorité: 02.06.1999 FR 9906964
(71) Demandeur: L'OREAL, 75008 Paris (FR)
(72) Inventeur: Tournilhac, Florence, 75011 Paris (FR); Lorant, Raluca, 94320 Thiais (FR)
(74) Mandataire: Rasson, Catherine

(57) **Abrégé**

La présente demande se rapporte à une composition sous forme d'émulsion huile-dans-eau, comprenant dans un milieu physiologiquement acceptable, une phase huileuse dispersée dans une phase aqueuse, caractérisée en ce qu'elle est exempte de tensioactif et en ce qu'elle contient des fibrilles de cellulose qui ont une longueur supérieure à 1 micron et un rapport longueur/diamètre supérieur à 30.

Les fibrilles de cellulose peuvent être des microfibrilles ou des nanofibrilles et être éventuellement modifiées.

La composition obtenue bien qu'exempte de tout tensioactif a une très bonne stabilité. Elle peut notamment constituer une composition cosmétique.

L'invention se rapporte aussi à l'utilisation de la dite composition, notamment pour le soin, le traitement, le maquillage ou le nettoyage de la peau, des lèvres, des cils et/ou des cheveux, ainsi que pour le soin des peaux sensibles ou sèches.

## Description

La présente demande se rapporte à une composition sous forme d'émulsion huile-dans-eau exempte de tensioactif et contenant des fibrilles de cellulose, et à l'utilisation de la dite composition, en particulier pour le soin, le traitement et/ou le maquillage de la peau du corps ou du visage, des cheveux, des cils et/ou des lèvres. La demande se rapporte aussi à l'utilisation de fibrilles de cellulose pour la stabilisation d'une émulsion huile-dans-eau exempte de tensioactif.

Pour diverses raisons liées en particulier à un meilleur confort d'utilisation (douceur, émollience et autres), les compositions cosmétiques actuelles se présentent le plus souvent sous la forme d'une émulsion du type huile-dans-eau (H/E) constituée d'une phase continue dispersante aqueuse et d'une phase discontinue dispersée huileuse ou d'une émulsion du type eau-dans-huile (E/H) constituée d'une phase continue dispersante huileuse et d'une phase discontinue dispersée aqueuse. Les émulsions H/E sont les plus demandées dans le domaine cosmétique du fait qu'elles comportent comme phase externe, une phase aqueuse, ce qui leur confère, lors de l'application sur la peau, un toucher plus frais, moins gras et plus léger que les émulsions E/H.

Les émulsions sont généralement stabilisées par des tensioactifs émulsionnants appropriés qui, grâce à leur structure amphiphile, se placent à l'interface huile/eau et stabilisent ainsi les gouttelettes dispersées. Ces émulsionnants présentent cependant l'inconvénient d'être pénétrants et potentiellement irritants pour la peau, les yeux et le cuir chevelu, notamment pour les sujets à peau sensible.

En outre, de telles émulsions peuvent avoir des propriétés cosmétiques et physico-chimiques insuffisantes (toucher huileux, instabilité dans le temps). Le fait d'augmenter le taux des tensioactifs ne résout pas généralement les problèmes mentionnés. La stabilité requise n'est pas toujours atteinte et les propriétés cosmétiques ne sont pas améliorées (toucher cireux, lourd, manque de fraîcheur à l'application). Par ailleurs, comme indiqué ci-dessus, il est aussi déconseillé d'utiliser un trop fort taux de tensioactif pour des raisons d'innocuité.

Une solution pour s'affranchir des phénomènes d'instabilité des émulsions H/E (crémage et déphasage) consiste à ajouter dans l'émulsion des agents épaississants dont la fonction est de créer, au sein de la phase aqueuse, une matrice gélifiée servant à figer les gouttelettes huileuses et assurant un maintien mécanique de l'ensemble de l'émulsion. Toutefois, cette solution présente l'inconvénient de ne pas permettre d'obtenir toutes les textures désirées et en particulier les textures légères, s'appliquant facilement et rapidement sur la peau sans laisser de film résiduel.

Par ailleurs, il a été envisagé de remplacer les tensioactifs par des polymères comportant dans leur chaîne une partie hydrophile et une partie hydrophobe constituée d'une chaîne grasse, tels que les copolymères d'alkyl-C₁₀-C₃₀-acrylate et d'acide acrylique ou méthacrylique, comme le produit "PEMULEN TR2" commercialisé par la société Goodrich. Cependant, ces polymères présentent l'inconvénient d'apporter un effet collant lors de l'application sur la peau et de ne pas permettre l'obtention d'une composition qui reste stable pendant une grande période de temps quand la quantité d'huile est trop importante.

L'objectif de l'invention est de pouvoir réaliser des émulsions huile-dans-eau stables ne contenant pas de tensioactif émulsionnant classiquement utilisé dans les émulsions H/E, présentant de bonnes propriétés cosmétiques sans avoir les inconvénients de l'art antérieur.

La demanderesse a découvert de façon inattendue que les fibrilles de cellulose permettaient de réaliser des émulsions huile-dans-eau stables bien qu'exemptes de tensioactif.

Certes, il est connu d'utiliser des fibrilles de cellulose dans les compositions cosmétiques. Toutefois, il n'a jamais été envisagé de les utiliser dans des émulsions H/E pour stabiliser ces émulsions en l'absence de tout tensioactif.

La présente invention concerne une composition sous forme d'émulsion huile-dans-eau comprenant dans un milieu physiologiquement acceptable, une phase huileuse dispersée dans une phase aqueuse, caractérisée par le fait qu'elle est exempte de tensioactif et qu'elle contient des fibrilles de cellulose qui ont une longueur supérieure à 1 micron et un rapport longueur/diamètre supérieur à 30.

L'invention a encore pour objet l'utilisation de fibrilles de cellulose pour la stabilisation d'une émulsion huile-dans-eau exempte de tensioactif.

La composition obtenue a une texture homogène et agréable à l'application. En outre, bien qu'exempte de tensioactif, elle reste stable dans le temps à température ambiante ou à des températures plus élevées. De plus, du fait de l'absence de tensioactif, elle présente l'avantage de ne pas être irritante pour les peaux particulièrement sensibles et de permettre, en outre, l'incorporation d'actifs thermosensibles car elle peut être fabriquée à température ambiante.

On entend ici par « milieu physiologiquement acceptable », un milieu compatible avec la peau, les lèvres, le cuir chevelu, les cils, les yeux et/ou les cheveux.

Par ailleurs, on entend dans la présente demande par « fibrilles de cellulose » aussi bien des nanofibrilles que des microfibrilles Ces fibrilles ont une longueur supérieure à 1 µm et de préférence allant de 5 à 40 µm et un rapport longueur/diamètre égal ou supérieur à 30. Le diamètre des fibrilles utilisées peut aller par exemple de 2 à 100 nm (0,002 à 0,1 µm).

Les fibrilles de cellulose utilisées selon l'invention sont de préférence amorphes, c'est-à-dire qu'elles présentent de préférence un taux de cristallinité inférieur ou égal à 50 %, et de préférence allant de 15 à 50 %.

En outre, les fibrilles de cellulose utilisées selon l'invention peuvent être obtenues soit par extraction mécanique ou chimique à partir de végétaux ou d'algues, soit par fermentation bactérienne. Par ailleurs, elles peuvent se présenter sous forme de matière sèche ou en dispersion notamment aqueuse.

Les fibrilles de cellulose utilisées selon l'invention peuvent se présenter telles quelles ou modifiées. Ainsi, elles peuvent être en mélange avec un additif et notamment avec de la cellulose carboxylée, comme décrit dans les documents WO-A-98/02486 et WO-A-98/02487. Elles peuvent être également sous forme modifiée, et par exemple elles peuvent être modifiées par des acides carboxyliques comme décrit par exemple dans le document EP-A-726356, et/ou être associées à un composé organique polyhydroxylé comme décrit par exemple dans le document FR-A-2,769,836.

Les fibrilles utilisées selon l'invention sont de préférence celles obtenues par mélange en milieu aqueux, des fibrilles de cellulose avec de la cellulose carboxylée, en particulier de la carboxyméthylcellulose, et séchage de ce mélange, comme décrit par exemple dans le document FR-A-2,769,836.

On peut notamment utiliser comme fibrilles de cellulose celles commercialisées sous les dénominations « cellulon » par la société Kelco, celle commercialisée sous la dénomination « Fibriliance » par la société Soliance et celles de la société Rhodia, notamment celle appelée ci-après « composé A », comprenant 85 % de nanofibrilles et 15 % de carboxyméthylcellulose et obtenues selon l'exemple 1 du document FR-A-2,769,836.

Les fibrilles de cellulose sont généralement introduites dans la phase aqueuse de l'émulsion.

La composition de l'invention peut contenir une quantité de fibrilles de cellulose, allant de 0,05 à 20 % de matière active en poids, de préférence de 0,1 à 10 % et mieux de 0,5 à 5 % en poids de matière active, par rapport au poids total de la composition.

La phase huileuse de la composition selon l'invention représente généralement de 10 à 40 % et de préférence de 15 à 30 % en poids par rapport au poids total de la composition.

La phase huileuse peut être constituée par tous les corps gras et notamment les huiles, classiquement utilisés dans les domaines cosmétique ou dermatologique.

Parmi les huiles utilisables dans l'émulsion de l'invention, on peut citer par exemple les huiles végétales telles que les huiles de jojoba, avocat, amande douce, abricot, maïs et la fraction liquide de beurre de karité ; les huiles minérales comme l'huile de vaseline et le polyisobutène hydrogéné ; les huiles de synthèse comme le palmitate d'éthyl-2 hexyle, le myristate d'isopropyle, l'isoparaffine hydrogénée, l'isononanoate d'isononyle, l'octanoate de cétéaryle ; les huiles de silicone volatiles ou non volatiles et les huiles fluorées. Les autres corps gras susceptibles d'être présents dans la phase huileuse peuvent être par exemple les acides gras, les alcools gras et les cires.

Selon un mode particulier de réalisation de l'invention, la composition de l'invention contient au moins une huile minérale telle que l'huile de vaseline et le polyisobutène hydrogéné. Selon un mode plus particulièrement préféré, il s'agit de l'huile de vaseline qui permet d'obtenir des compositions ayant une texture particulièrement agréable.

Ainsi, selon un mode particulier de réalisation de l'invention, la composition selon l'invention comprend au moins 5 % en poids, de préférence au moins 10 % en poids et mieux 15 % en poids d'huile minérale, et notamment d'huile de vaseline, par rapport au poids total de la composition.

La phase aqueuse de la composition de l'invention constitue en général de 60 à 90 % et de préférence de 70 à 85 % en poids par rapport au poids total de la composition.

De façon connue, les compositions de l'invention peuvent contenir des adjuvants habituels dans les domaines considérés, tels que des actifs hydrophiles ou lipophiles, des conservateurs, des gélifiants, des antioxydants, des parfums, des solvants, des charges et notamment des charges matifiantes ou des nacres, des filtres, des matières colorantes (pigments ou colorants solubles), des agents basiques ou acides et encore des vésicules lipidiques. Ces adjuvants sont utilisés dans les proportions habituelles dans le domaine cosmétique, et par exemple de 0,01 à 30 % du poids total de l'émulsion, et ils sont, selon leur nature, introduits dans la phase aqueuse ou dans la phase huileuse de l'émulsion, ou encore dans des vésicules. Ces adjuvants ainsi que leurs concentrations doivent être tels qu'ils ne modifient pas la propriété recherchée pour l'émulsion de l'invention.

Comme actifs, on peut citer par exemple les hydratants tels que les polyols comme la glycérine et le sorbitol ; les agents kératolytiques ; les dépigmentants ; les amincissants, et tout actif approprié pour le but final de la composition.

Selon la fluidité de la composition que l'on souhaite obtenir, on peut y ajouter un ou plusieurs gélifiants hydrophiles ou lipophiles. Comme gélifiants hydrophiles, on peut citer par exemple les dérivés cellulosiques tels que la carboxyméthylcellulose ; les gommes polysaccharides et leurs dérivés (gomme de xanthane, carboxyméthyl-hydroxypropyl guar) ; les protéines ; les polymères acryliques et vinyliques ; les polymères associatifs comme les polyuréthanes, les polyacryliques ou polyacrylamides et les polymères naturels modifiés. Comme gélifiants lipophiles, on peut citer les copolymères acryliques tels que celui vendu sous la dénomination « Structure O » par la société National Starch (nom CTFA : acrylates copolymer) ; les dérivés de guar modifiés hydrophobes ; les dérivés de polyéthylène ; les dérivés élastomères tri-blocs styrène // butylène et/ou éthylène // styrène ; les argiles telles que la bentone. On peut aussi utiliser un mélange de ces gélifiants.

Ces gélifiants lorsqu'ils sont présents, sont généralement utilisés à des concentrations allant de 0,1 à 10 %, de préférence de 0,1 à 5 % et mieux de 0,1 à 3 % en poids de matière active par rapport au poids total de la composition.

Les compositions, objets de l'invention, trouvent leur application dans un grand nombre de traitements notamment cosmétiques et peuvent ainsi constituer une composition cosmétique, notamment pour le traitement, la protection, le soin, le démaquillage et/ou le nettoyage de la peau, des lèvres et/ou des cheveux, et/ou pour le maquillage de la peau des lèvres, des cils et/ou du corps.

Les compositions selon l'invention peuvent par exemple être utilisées comme produits de soin, de démaquillage et/ou de nettoyage pour le visage sous forme de crèmes ou de laits ou comme produits de maquillage (peau, cils et lèvres) par incorporation de pigments ou de colorants, par exemple comme fonds de teint ou comme mascaras.

Aussi, l'invention a pour objet l'utilisation cosmétique de la composition telle que définie ci-dessus pour le traitement, la protection, le soin, le démaquillage et/ou le nettoyage de la peau, des lèvres et/ou des cheveux, et/ou pour le maquillage de la peau, des lèvres, des cils et/ou du corps.

L'invention a aussi pour objet un procédé de traitement cosmétique de la peau, y compris du cuir chevelu, des cheveux, des cils et/ou des lèvres, caractérisé par le fait qu'on applique sur la peau, les cheveux, les cils et/ou les lèvres, une composition telle que définie ci-dessus.

Du fait que la composition est exempte de tensioactif, elle est particulièrement bien tolérée par les sujets ayant une peau sensible. Par ailleurs, elle est aussi adaptée pour les peaux sèches.

L'invention a donc encore pour objet l'utilisation de la composition telle que définie ci-dessus pour la fabrication d'une composition destinée au soin des peaux sèches et/ou sensibles.

Les exemples qui suivent permettront de mieux comprendre l'invention, sans toutefois présenter un caractère limitatif. Les quantités indiquées sont en % en poids, sauf mention contraire.

### Exemple 1 : Crème

| *Phase aqueuse :* | |
|---|---|
| Nanofibrille de cellulose (Composé A de la société Rhodia) | 1 % |
| Conservateurs | 0,3 % |
| Eau déminéralisée | qsp 100 % |

| *Phase huileuse :* | |
|---|---|
| Huile de vaseline | 20 % |

Mode opératoire : La phase aqueuse est homogénéisée par dispersion à l'aide de l'homogénéisateur sous pression. Puis on y ajoute la phase huileuse et on effectue encore deux passages en homogénéisation sous pression.

On obtient une crème qui, à l'application sur la peau, est fraîche comme un lait tout en étant nutritive. Cette crème est particulièrement adaptée pour le soin des peaux sèches et sensibles.

### Exemple 2 : : Crème nutritive pour peaux sensibles

| *Phase aqueuse :* | |
|---|---|
| Conservateurs | 0,3 % |
| Glycérol | 5 % |
| Microfibrilles de cellulose (Cellulon PC de la société Kelco) | 1,7 % |
| Carboxyméthylcellulose | 0,3 % |
| Eau déminéralisée | qsp 100 % |

| *Phase huileuse :* | |
|---|---|
| Huile de noyaux d'abricots | 10 % |
| Polyisobutène hydrogéné | 5 % |
| Acrylates copolymer (Structure O de la société National Starch) | 1 % |

Mode opératoire : La phase aqueuse est homogénéisée par simple agitation, puis on y disperse sous agitation la phase huileuse préalablement homogénéisée à 70°C.

On obtient une crème particulièrement bien tolérée par les sujets à peau sensible.

### Exemple 3 : Fond de teint

| *Phase aqueuse :* | |
|---|---|
| Microfibrille de cellulose (Composé A de la société Rhodia) | 1 % |
| Conservateurs | 0,3 % |
| Eau déminéralisée | qsp 100 % |

| *Phase huileuse :* | |
|---|---|
| Huile de vaseline | 20 % |
| Oxydes de fer | 4 % |
| Oxyde de titane | 1 % |

Mode opératoire : On prépare la phase aqueuse sous agitation dans un homogénéisateur, puis on y ajoute la phase huileuse préalablement préparée par broyat des pigments dans l'huile.

On obtient un fond de teint ayant l'aspect d'une crème, facile à étaler et à uniformiser comme un fond de teint fluide.

## Revendications

1. Composition sous forme d'émulsion huile-dans-eau comprenant dans un milieu physiologiquement acceptable, une phase huileuse dispersée dans une phase aqueuse, caractérisée par le fait qu'elle est exempte de tensioactif et qu'elle contient des fibrilles de cellulose qui ont une longueur supérieure à 1 micron et un rapport longueur/diamètre supérieur à 30.

2. Composition selon la revendication 1, caractérisée en ce que les fibrilles de cellulose ont une longueur allant de 5 à 40 µm.

3. Composition selon la revendication 1 ou 2, caractérisée en ce que les fibrilles de cellulose ont un diamètre allant de 2 à 100 nm.

4. Composition selon l'une quelconque des revendications précédentes, caractérisée en ce que les fibrilles de cellulose sont en mélange avec de la cellulose carboxylée et/ou sont modifiées par des acides carboxyliques et/ou sont associées à un composé organique polyhydroxylé.

5. Composition selon l'une quelconque des revendications précédentes, caractérisée en ce que les fibrilles de cellulose sont celles obtenues par mélange, en milieu aqueux, des fibrilles de cellulose avec de la cellulose carboxylée et séchage de ce mélange.

6. Composition selon l'une quelconque des revendications précédentes, caractérisée en ce que les fibrilles de cellulose sont présentes en une quantité allant de 0,05 à 20 % de matière active en poids par rapport au poids total de la composition.

7. Composition selon l'une quelconque des revendications précédentes, caractérisée en ce que la phase huileuse représente de 10 à 40 % en poids par rapport au poids total de la composition.

8. Composition selon l'une quelconque des revendications précédentes, caractérisée en ce que la phase huileuse comprend au moins une huile minérale.

9. Composition selon la revendication précédente, caractérisée en ce que la phase huileuse comprend de l'huile de vaseline et/ou du polyisobutène hydrogéné.

10. Composition selon la revendication 8 ou 9, caractérisée en ce que la phase huileuse comprend au moins 5 % en poids d'huile minérale, par rapport au poids total de la composition.

11. Composition selon l'une quelconque des revendications précédentes, caractérisée en ce qu'elle comprend au moins un gélifiant hydrophile ou lipophile.

12. Composition selon l'une quelconque des revendications précédentes, caractérisée en ce qu'elle constitue une composition cosmétique.

13. Utilisation cosmétique de la composition selon l'une quelconque des revendications 1 à 12, pour le traitement, la protection, le soin, le démaquillage et/ou le nettoyage de la peau, des lèvres et/ou des cheveux, et/ou pour le maquillage de la peau, des lèvres, des cils et/ou du corps.

14. Procédé de traitement cosmétique de la peau, des cheveux, des cils et/ou des lèvres, caractérisé en ce qu'on applique sur la peau, les cheveux, les cils et/ou les lèvres, une composition selon l'une quelconque des revendications 1 à 12.

15. Utilisation de la composition selon l'une quelconque des revendications 1 à 12, pour la fabrication d'une composition destinée au soin des peaux sèches et/ou sensibles.

16. Utilisation de fibrilles de cellulose ayant une longueur supérieure à 1 micron et un rapport longueur/diamètre supérieur à 30, pour la stabilisation d'une émulsion huile-dans-eau exempte de tensioactif.
